# EUROPÄISCHE PATENTSCHRIFT

(11) **EP 2 315 557 B1**
(45) Veröffentlichungstag und Bekanntmachung des Hinweises auf die Patenterteilung: **26.09.2012**
(21) Anmeldenummer: 09776106.8
(22) Anmeldetag: 21.08.2009
(51) Int. Cl.: A61F 2/06

(54) **BLUTGEFÄßANSCHLUSS FÜR EINE GEFÄSSPROTHESE**
BLOOD VESSEL ATTACHMENT FOR A VASCULAR PROSTHESIS
ÉLÉMENT DE CONNEXION DE VAISSEAU SANGUIN POUR PROTHÈSE VASCULAIRE

(30) Priorität: 23.08.2008 DE 102008039523
(43) Veröffentlichungstag der Anmeldung: 04.05.2011
(73) Patentinhaber: S&C Systems GmbH, 23558 Lübeck (DE)
(72) Erfinder: GERHARDT, Dietmar, 24848 Kropp (DE)
(74) Vertreter: Hemmer, Arnd
(86) Internationale Anmeldenummer: PCT/DE2009/001174
(87) Internationale Veröffentlichungsnummer: WO 2010/022705

(56) Entgegenhaltungen:
- WO-A-00/24339
- WO-A-2007/140566
- US-A- 4 190 909
- US-A- 4 368 736

## Beschreibung

Die Erfindung betrifft einen Blutgefäßanschluss für eine Gefäßprothese.

Es ist üblich, verletzungs- oder krankheitsbedingt geschädigte Abschnitte von Blutgefäßen, insbesondere Arterien z. B. bei einem Aneurysma, durch Gefäßprothesen zu ersetzen. Diese Gefäßprothesen sind typischerweise schlauchförmig aus körpervertröglichen Kunststoffen wie beispielsweise Polytetrafluorethylen ausgebildet, wobei ihre Querschnittsabmessungen im Wesentlichen den Querschnittsmaßen der zu ersetzenden Blutgefäßabschnitte entsprechen.

Die Verbindung der Gefäßprothese mit den offenen Enden des Blutgefäßes erfolgt bislang durch Vernähen. Dies ist verhältnismäßig zeitaufwendig und daher sowohl für den Operateur als auch für den Patienten belastend. Des Weiteren besteht die Gefahr, dass es im vernähten Bereich in Folge von Undichtigkeiten der durch die Einstichkanäle perforierten Gefäßwand zu einem Aneurysma kommt oder dort an einem Querschnittsübergang ein Thrombus entsteht.

Aus WO 00/24339 A ist eine Vorrichtung zur Verbindung zweier Gefäße mit den im Oberbegriff des Anspruchs 1 angegebenen Merkmalen bekannt. Nachteilig bei dieser Vorrichtung ist, dass die Montage ein erhebliches Geschick verlangt und sich im Inneren des Strömungspfades Thromben bilden können.

Vor diesem Hintergrund liegt der Erfindung die Aufgabe zugrunde, eine Verbindung eines Blutgefäßes mit einer Gefäßprothese zu schaffen, bei der die Gefahr, dass eine der oben genannten medizinischen Komplikationen auftritt, gegenüber einer genähten Verbindung verringert ist. Darüber hinaus soll eine Gefäßprothese mit dieser Verbindung deutlich schneller an einem Blutgefäß befestigt werden können.

Gelöst wird diese Aufgabe mit einem Blutgefäßanschluss für eine Gefäßprothese mit den in Anspruch 1 angegebenen Merkmalen. Vorteilhafte Weiterbildungen dieses Blutgefäßanschlusses ergeben sich aus den Unteransprüchen, der nachfolgenden Beschreibung sowie den Zeichnungen.

Zur Verbindung eines Blutgefäßes mit einer Gefäßprothese sieht die Erfindung einen Blutgefäßanschluss mit einem Anschlussteil vor. Das Anschlussteil ist abschnittsweise in das nach dem Entfernen eines geschädigten Gefäßabschnitts entstehende offene Ende des Blutgefäßes einführbar und bildet einen Strömungskanal von dem Blutgefäß zu der ebenfalls an dem Anschlussteil angeschlossenen Gefäßprothese. Zur Befestigung des Blutgefäßes an dem Anschlussteil weist der Blutgefäßanschluss Klemmmittel auf. Mit diesen Klemmmitteln wird auf die Außenseite des Blutgefäßes und zweckmäßigerweise über dessen gesamten Umfang eine Kraft ausgeübt, die das Blutgefäß kraftschlüssig und/oder formschlüssig an dem Anschlussteil festlegt. Gegebenenfalls kann eine auf diese Weise geschaffene feste Verbindung des Blutgefäßes mit der Gefäßprothese dadurch verbessert werden, dass das Blutgefäß zusätzlich mit einem gewebeverträglichen Klebstoff mit dem Anschlussteil und/oder den Klemmmitteln verklebt wird.

Vorteilhafterweise ist also mit dem erfindungsgemäßen Blutgefaßanschluss eine Verbindung einer Gefäßprothese mit einem Blutgefäß möglich, ohne das Implantat mit dem Gefäß vernähen zu müssen. Die Dauer des operativen Eingriffs beim Einsetzen der Gefäßprothese wird so erheblich verkürzt. Dies ist insbesondere dann von Vorteil, wenn die Gefäßprothese in solche Blutgefäße eingesetzt werden soll, die lebenswichtige Organe mit Blut versorgen. Die Blutversorgung dieser Organe wird bei Verwendung des erfindungsgemäßen Blutgefäßanschlusses deutlich kürzer unterbrochen.

Ein weiterer Vorteil des erfindungsgemäßen Blutgefößanschlusses ist darin zu sehen, dass, anders als beim bislang üblichen Vernähen des Blutgefäßes mit der Gefäßprothese, der gesunde Teil des Blutgefäßes nicht beschädigt wird. Dementsprechend besteht bei Einsatz des erfindungsgemäßen Blutgefäßanschlusses eine deutlich geringere Gefahr, dass sich in an die Gefäßprothese anschließenden Gefäßabschnitten ein Aneurysma bildet.

Der Abschnitt des Anschlussteils, der in das Blutgefäß eingeführt werden soll, ist rohrförmig ausgebildet, wobei dieser rohrförmige Abschnitt des Anschlussteils typischerweise einen Außenquerschnitt aufweist, der im Wesentlichen mit dem Innenquerschnitt des Blutgefäßes korrespondiert. Zur Befestigung des Blutgefäßes an dem darin eingeführten Abschnitt des Anschlussteils weist der Blutgefäßanschluss ein hülsenförmiges Klemmbauteil auf, das zur Anordnung außenseitig des rohrförmigen Abschnitts des Anschlussteils vorgesehen ist. D. h., ähnlich der Verwendung einer Schlauchschelle wird das Blutgefäß bei dieser Ausgestaltung über seinen gesamten Umfang von dem Klemmbauteil auf den in dem Blutgefäß befindlichen Abschnitt des Anschlussteils gepresst. Dies stellt sicher, dass im Bereich der Verbindung des Blutgefäßes mit dem Blutgefäßanschluss keine unerwünschten Blutverluste auftreten können.

Eine besonders gute Dichtheit dieser Verbindung kann dann erzielt werden, wenn das hülsenförmige Klemmbauteil das Blutgefäß in dem Abschnitt, in dem es das eingeführte Anschlussteil überlappt, im Wesentlichen über die gesamte Länge dieses Abschnitts umhüllt. Demzufolge klemmt das Klemmbauteil das Blutgefäß an dem Anschlussteil bevorzugt in einem Bereich fest, der sich in Längsrichtung des Blutgefäßes bzw. des Anschlussteils im Wesentlichen von dem distalen Ende des Anschlussteils, das heißt dem Ende des Anschlussteils, das in das Blutgefäß eingeführt ist, bis zu dem offenen Ende des Blutgefäßes erstreckt. Dies ist insofern vorteilhaft, als das Blutgefäß mit einer ausreichend großen Klemmkraft auf dem Anschlussteil gehalten wird, ohne dass eine Schädigung der Blutgefäßwandung aufgrund dieser Klemmkraft zu befürchten ist, da die Flächenpressung auf das Blutgefäß aufgrund des so geschaffenen großen Überlappungsbereichs bzw. Anlagebereichs des Klemmbauteils auf dem Blutgefäß vergleichsweise gering ist. Ein weiterer Vorteil ist darin zu sehen, dass das Klemmbauteil dadurch, dass es an dem Blutgefäß in dem gesamten Bereich zur Anlage kommt, in dem das Blutgefäß das Anschlussteil überlappt, sicherstellt, dass sich zwischen dem Blutgefäß und dem Anschlussteil keine Toträume bilden können, in denen sich ansonsten Blutgerinnsel festsetzen könnten.

Bevorzugt ist das Klemmbauteil zweiteilig ausgebildet. Zweckmäßigerweise erfolgt die Teilung des Klemmbauteils hierbei in einer Ebene parallel zur Längsachse des Klemmbauteils und besonders vorteilhaft in einer gemeinsamen Ebene mit der Längsachse des Klemmbauteils, so dass das Klemmbauteil quasi in zwei Hälften geteilt ist. Dementsprechend besteht das Klemmbauteil bei dieser Ausgestaltung aus zwei im Wesentlichen schalenförmigen Teilen, die in einfacher Weise an der Außenseite des Blutgefäßes angeordnet und dort unter Bildung einer festen Verbindung zusammengefügt werden können.

In diesem Zusammenhang ist bevorzugt vorgesehen, dass die Teile des Klemmbauteils zur Bildung einer Rastverbindung ausgebildet sind. Die beiden Teile des Klemmbauteils können also vorzugsweise im Bereich ihrer Fügekanten mit dem jeweils anderen Teil des Klemmbauteils Rastmittel aufweisen, die miteinander derart in Eingriff gebracht werden können, dass nach dem Einrasten dieser Rastmittel eine form- und/oder kraftschlüssige Verbindung der beiden Teile des Klemmbauteils entsteht.

Zur Befestigung der beiden Teile des Klemmbauteils kann vorteil hafterweise an dem Klemmbauteil zumindest eine sich um den Außenumfang des Klemmbauteils erstreckende Nut ausgebildet sein. D. h., die beiden Teile des Klemmbauteils können jeweils an ihrer Außenseite mindestens eine Nut aufweisen, die im zusammengefügten Zustand der beiden Teile eine um den gesamten Umfang des Klemmbauteils verlaufende Nut ergeben. Diese Nut kann zur Aufnahme eines Befestigungsfadens dienen, der die beiden Teile des Klemmbauteils, nachdem die Enden des Befestigungsfadens miteinander verknotet worden sind, fest zusammen hält. Eine besonders sichere Verbindung der beiden Teile des Klemmbauteils ist dann möglich, wenn diese Teile sowohl mit an diesen ausgebildeten Rastmitteln als auch mit einem, vorzugsweise mit mehreren in der oben beschriebenen Weise angeordneten Befestigungsfäden miteinander verbunden sind. Für mehrere Befestigungsfäden werden entsprechend mehrere Nuten am Außenumfang des Klemmbauteils vorgesehen.

Eine weitere bevorzugte Ausgestaltung des erfindungsgemäßen Blutgefäßanschlusses sieht vor, dass die beiden Teile des Klemmbauteils mittels eines Scharniers verbunden sind. D. h., die beiden Teile des Klemmbauteils können an zwei einander gegenüberliegenden Fügekanten mittels des Scharniers gelenkig und bevorzugt nicht lösbar verbunden sein, wobei die beiden Teile jeweils an ihrer anderen Fügekante lösbar, vorzugsweise mit Rastmitteln miteinander verbindbar sind. Vorteilhaft an dieser Ausgestaltung ist es, dass das Klemmbauteil als einteiliges Bauteil deutlich einfacher als bei einer zweiteiligen Ausgestaltung mit zwei separaten Teilen um das Blutgefäß herum angeordnet und dort befestigt werden kann.

Um ein Verrutschen des Klemmbauteils in axialer Richtung relativ zu dem Anschlussteil zu verhindern, sind das Klemmbauteil und das Anschussteil vorteilhafterweise formschlüssig miteinander verbindbar. So weist das Klemm bauteil bevorzugt zumindest einen radial nach innen gerichteten Vorsprung auf, der zum Eingriff in zumindest eine an der Außenseite des Anschlussteils ausgebildete Ausnehmung vorgesehen ist. Alternativ oder zusätzlich kann auch an dem Anschlussteil zumindest ein sich radial nach außen erstreckender Vorsprung vorgesehen sein, der in zumindest eine an der Innenseite des Klemmbauteils ausgebildete Ausnehmung eingreift. Um das Blutgefäß bei der Bildung eines solchen Formschlusses nicht zu verletzen, ist das Klemmbauteil zweckmäßig derart dimensioniert, dass es das offene Ende des Blutgefäßes in axialer Richtung überragt, wobei der Formschluss zwischen dem Klemmbauteil und dem Anschlussteil in diesem, das Blutgefäß überragenden Bereich hergestellt wird. D. h. das Klemmbauteil weist einen Klemmabschnitt zum Klemmen des Blutgefäßes und einen sich in axialer Richtung anschließenden Verbindungsabschnitt zum Eingriff mit dem Anschlussteil auf.

Ein Verrutschen des Blutgefäßes in axialer Richtung relativ zu dem Anschlussteil und dem Klemmbauteil wird vorzugsweise dadurch verhindert, dass außenseitig des in das Blutgefäß einführbaren Abschnitts des Anschlussteils zumindest ein radial nach außen gerichteter Vorsprung ausgebildet ist, der sich vorzugsweise um den gesamten Umfang des Anschlussteils erstreckt. Dieser Vorsprung drückt sich dann, wenn das Blutgefäß zwischen dem Anschlussteil und dem Klemmbauteil verklemmt wird, unter Verdrängung von Blutgefäßgewebe in die Wandung des Blutgefäßes quer zu einer möglichen axialen Bewegungsrichtung des Blutgefäßes ein. Zweckmäßigerweise ist der Vorsprung so dimensioniert und ausgebildet, dass mit ihm die Wandung des Blutgefäßes nicht perforiert werden kann. Durch einen oder mehrere solche Vorsprünge, welche eine Querrillung bilden, kann der Halt des Blutgefäßes bei verringertem Anpressdruck durch das Klemmbauteil verbessert werden.

Das Anschlussteils ist bevorzugt derart ausgebildet, dass sich sein Außenquerschnitt im Bereich des röhrformigen Abschnitts in Richtung dessen freien Endes verjüngt. Hierbei ist zweckmäßigerweise vorgesehen, dass das Anschlussteil zumindest in dem Bereich, in dem es von dem Blutgefäß überlappt wird, einen konstanten Innenquerschnitt aufweist, der im Wesentlichen dem Innenquerschnitt des Blutgefäßes entspricht und sich lediglich die Wandstärke des Anschlussteils in Richtung des distalen Endes des Anschlussteils bevorzugt kontinuierlich verringert. Dies ist insofern vorteilhaft, als sich hierdurch beim Übergang von dem Anschlussteil zu dem daran angeschlossenen Blutgefäß allenfalls eine sehr kleine und vorzugsweise nahezu keine Querschnittsänderung des Strömungspfads in dem Blutgefäß ergibt, so dass an dieser Stelle entstehende Thromben weitesgehend ausgeschlossen werden können. Ein weiterer Vorteil dieser Ausgestaltung ist darin zu sehen, dass sie das Einführen des Anschlussteils in das Blutgefäß erleichtert.

Erfindungsgemäß ist weiter vorgesehen, dass sich ein den Strömungskanal durch das Anschlussteil bildender Hohlraum des Anschlussteils in einem Abschnitt, der sich an den rohrförmigen in das Blutgefäß einführbaren Abschnitt anschließt, absatzförmig erweitert. Hierbei weist der Blutgefäßanschluss eine Befestigungshülse zum Fixieren der Gefäßprothese auf, die in den erweiterten Abschnitt des Anschlussteils einschraubbar ist. Dazu ist am Innenumfang des erweiterten Abschnitts ein Innengewinde und am Außenumfang der Befestigungshülse ein korrespondierendes Außengewinde ausgebildet. So kann die Befestigungshülse und damit die Gefäßprothese in einfacher Weise an dem Anschlussteil befestigt werden. Vorteilhaft kann diese Befestigung bereits vor dem operativen Eingriff zum Einsetzen der Gefäßprothese in einem Blutgefäß erfolgen, was die Dauer dieses Eingriffs weiter verkürzt.

Zweckmäßigerweise sind der erweiterte Abschnitt des Anschlussteils und die darin anschraubbare Befestigungshülse in radialer Richtung so dimensioniert, dass die Innenwandung der mittels der Befestigungshülse an dem Anschlussteil angeschlossenen Gefößprothese, deren Innenquerschnitt typischerweise im Wesentlichen mit dem Innenquerschnitt des Blutgefäßes übereinstimmt, mit der Innenwandung des von dem Anschlussteil gebildeten Strömungskanals fluchtet, sodass das Blutgefäß, das Anschlussteil und die Gefäßprothese einen im Wesentlichen absatzfreien, glatten Strömungskanal mit konstanten Innenquerschnitt bilden, um eine Embolus-Bildung innerhalb des erfindungsgemäßen Blutgefäßanschlusses zu verhindern.

Die Gefäßprothese kann direkt an der Befestigungshülse befestigt sein. Daneben ist es auch möglich, die Gefäßprothese mit der Befestigungshülse in dem Anschlussteil zu verklemmen. In diesem Zusammenhang ist eine Ausgestaltung bevorzugt, bei der die Befestigungshülse in axialer Richtung derart dimensioniert ist, dass eine durch die Befestigungshülse geführte Gefäßprothese, die das im eingeschraubten Zustand in das Anschlussteil eingreifende Ende der Befestigungshülse überlappt, zwischen diesem Stirnende der Befestigungshülse und einem stirnseitigen Absatz des Anschlussteils verklemmbar ist. Bei dieser Ausgestaltung ist zweckmäßigerweise die Wandung der Befestigungshülse im Bereich ihres in das Anschlussteil eingreifenden Endes von der außenseitig darum angeordneten Wandung des Anschlussteils beabstandet, so dass in diesem Bereich zwischen den Wandungen des Anschlussteils und der Befestigungshülse ein Zwischenraum ausgebildet ist, der zur Aufnahme des die Befestigungshülse überlappenden Abschnitts der Gefäßprothese dienen kann. D. h. die Befestigungshülse weist in einem axialen Bereich einen kleineren Außendurchmesser als der Innendurchmesser des erweiterten Abschnitts auf. In einem sich axial anschließenden Bereich weist die Befestigungshülse einen Außendurchmesser entsprechend dem Innendurchmesser des erweiterten Abschnitts auf. Dort ist bevorzugt das Gewinde ausgebildet.

Um ein Verwachsen des Blutgefäßanschlusses mit dem Blutgefäß zu erleichtern, weisen der rohrförmige Abschnitt des Anschlussteils und das Klemmbauteil jeweils über den Umfang verteilt, eine Vielzahl von Durchbrechungen auf. Die Durchbrechungen erstrecken sich als Öffnungen in radialer Richtung durch die Wandung vom Anschlussteil und/oder Klemmbauteil hindurch. Besonders bevorzugt sind sie als sich axial erstreckende Langlöcher ausgebildet. Die Durchbrechungen können auch dazu dienen, ein Absterben des Blutgefäßgewebes in dem Bereich, in dem das Blutgefäß zwischen dem Anschlussteil und dem Klemmbauteil verklemmt ist, zu verhindern, indem die Blutzufuhr durch die Durchbrechungen sichergestellt wird.

Die einzelnen Bauteile des Blutgefäßanschlusses sind bevorzugt aus einem gewebeverträglichen Kunststoff hergestellt. Solche Kunststoffe haben den Vorteil, dass die einzelnen Bauteile vergleichsweise einfach und preiswert mit den in der Kunststoffverarbeitungstechnik verwendeten Gießverfahren hergestellt werden können. Vorzugsweise ist der erfindungsgemäße Blutgefäßanschluss aus Polyethylenterephthalat ausgebildet. Allerdings ist auch eine teilweise oder vollständige Ausgestaltung aus Metall denkbar.

Nachfolgend wird die Erfindung anhand eines in den Zeichnungen dargestellten Ausführungsbeispiels näher erläutert. Darin zeigt:
- Fig. 1: einen Blutgefäßanschluss für eine Gefäßprothese in perspektivischer Explosionsdarstellung und

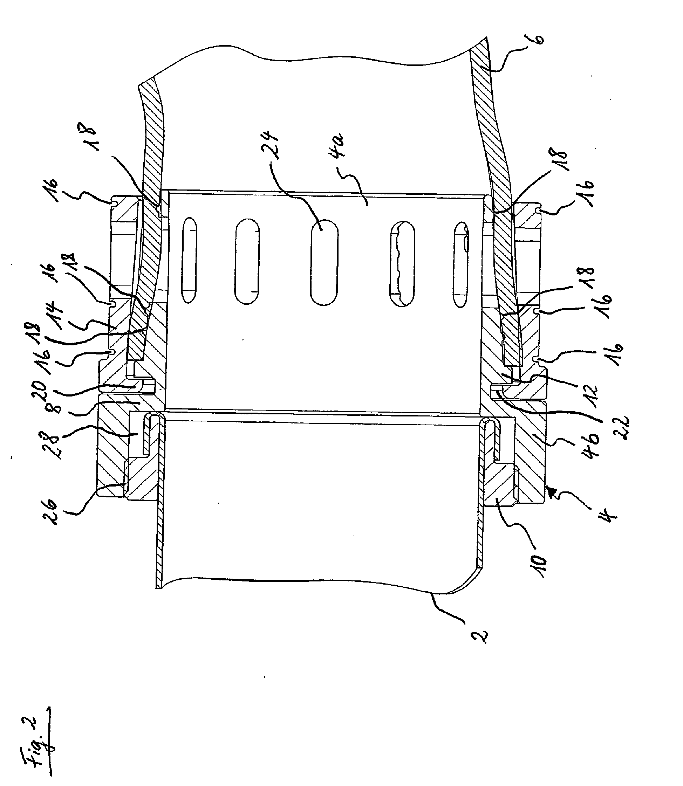

- Fig. 2: den Blutgefäßanschluss nach Fig. 1 im zusammengebauten Zustand in einem Längsschnitt.

Der erfindungsgemäße Blutgefäßanschluss für eine Gefäßprothese 2 weist ein Anschlussteil 4 auf. An diesem Anschlussteil 4 ist die Gefäßprothese 2 anschließbar. Darüber hinaus ist das Anschlussteil 4 in ein offenes Ende eines Blutgefäßes 6 einführbar. Das Anschlussteil 4 ist innen durchgehend hohl und bildet auf diese Weise einen axialen Strömungskanal von einem an dem Anschlussteil 4 angeschlossenen Blutgefäß 6 zu der ebenfalls an dem Anschlussteil 4 angeschlossenen Gefäßprothese 4.

Das Anschlussteil 4 weist zwei Abschnitte 4a und 4b auf, die sich hinsichtlich ihres Außenquerschnitts unterscheiden. So ist an dem Anschlussteil 4 ein Absatz 8 ausgebildet, an dem sich der Außendurchmesser des Abschnitts 4b gegenüber dem Außendurchmesser des Abschnitts 4a vergrößert. Korrespondierend hierzu vergrößert sich an dem Absatz 8 auch der den Strömungskanal durch das Anschlussteil 4 bildende Hohlraum, d. h. der Innendurchmesser. Der erweiterte Hohlraum im Bereich des Abschnitts 4b des Anschlussteils 4 dient zur Aufnahme einer Befestigungshülse 10, mit der die Gefäßprothese 2 an dem Anschlussteil 4 festgelegt wird.

Der Abschnitt 4a des Anschlussteils 4 dient dazu, in das Blutgefäß 6 eingeführt zu werden und so eine Strömungsverbindung von dem Blutgefäß 6 zu dem Blutgefäßanschluss und der daran angeschlossenen Gefäßprothese 2 zu schaffen. Entsprechend korrespondiert der Außenquerschnitt im Bereich des Abschnitts 4a im Wesentlichen mit dem Innenquerschnitt des anzuschließenden Blutgefäßes 6, wobei sich der Außenquerschnitt des Abschnitts 4a ausgehend von einem an der Außenseite des Abschnitts 4a ausgebildeten ringförmigen Vorsprung 12, der als Anschlag für das offene Ende des Blutgefäßes 6 dient, in Richtung des freien Endes des Abschnitts 4a verjüngt. Hierdurch verringert sich auch die Wandstärke des Anschlussteils 4 im Bereich des Abschnitts 4a, so dass das in das Blutgefäß 6 eingeführte Ende des Abschlussteils 4 in dem Blutgefäß 6 ein im Hinblick auf ein mögliche Trombenbildung vernachlässigbares Hindernis darstellt. Des weiteren erleicht die Querschnittsverjüngung im Bereich es Abschnitts 4a das Einführen des Anschlussteils 4 in das Blutgefäß 6. In dem Abschnitt 4a weist der von dem Anschlussteil 4 gebildete Strömungskanal einen konstanten Innenquerschnitt auf, der im Wesentlichen dem Innenquerschnitt des an dem Anschlussteil 4 anzuschließenden Blutgefäßes 6 entspricht, sodass sich ein nahezu absatzfreier Übergang von dem Blutgefäß 6 zu dem von dem Anschlussteil 4 im Abschnitt 4a gebildeten Strömungskanal ergibt.

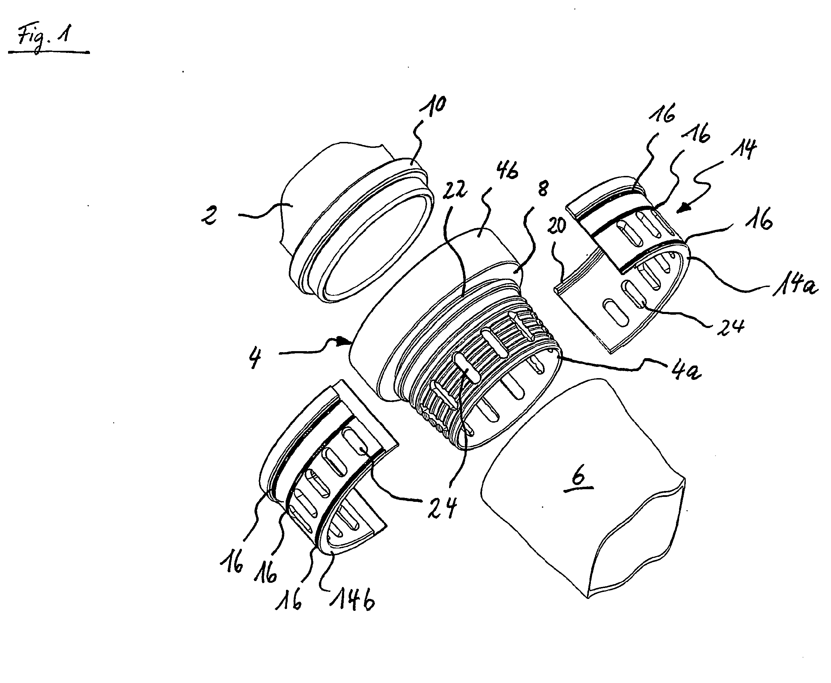

Zum kraftschlüssigen Festlegen des an der Außenseite des Abschnitts 4a anliegenden Blutgefäßes 6 an dem Anschlussteil 4 ist ein hülsenförmiges Klemmbauteil 14 vorgesehen. Das Klemmbauteil 14 ist zweiteilig ausgebildet (siehe Fig. 1) und weist zwei im Querschnitt halbkreisförmige schalenförmige Teile 14a und 14b auf, die in einfacher Weise zusammengefügt und beispielsweise mittels einer Rastverbindung (hier nicht gezeigt) miteinander fest verbunden werden können. Die Länge der beiden Teile 14a und 14b des Klemmbauteils 14 ist größer als die Länge des Bereichs, in dem das Blutgefäß 6 den Abschnitt 4a des Anschlussteils 4 überlappt.

Zur Sicherung der Verbindung der Teile 14a und 14b des Klemmbauteils 14 sind an der Außenseite der Teile 14a und 14b des Klemmbauteils 14 mehrere in Umfangsrichtung verlaufende Nuten 16 ausgebildet und derart angeordnet, dass sie jeweils im zusammengebauten Zustand des Klemmbauteils 14 ringförmig um den Umfang des Klemmbauteils 14 verlaufende Nuten 16 ergeben. In diesen Nuten 16 können nicht dargestellte Befestigungsfäden geführt sein, die nach dem Zusammenknoten der freien Enden dieser Befestigungsfäden ebenfalls das Klemmbauteil 14 zusammenhalten.

Um ein Verrutschen des Blutgefäßes 6 auf dem Abschnitt 4a in axialer Richtung des Anschlussteils 4 zu verhindern, sind an der Außenseite des Abschnitts 4a mehrere ringförmige Vorsprünge 18 ausgebildet, die sich in das Blutgefäß 6 unter Verdrängung von Blutgefäßgewebe eindrücken. Die Vorsprünge 18 sind abgerundet ausgebildet und vergleichsweise kurz, um eine Verletzung des Blutgefäßgewebes zu verhindern.

Ein Verrutschen des Klemmbauteils 14 in axialer Richtung des Anschlussteils 4 verhindert ein jeweils an einem axialen Ende der Klemmbauteile 14a und 14b an deren Innenseite ausgebildeter und sich über den gesamten Innenumfang erstreckender Vorsprung 20. Dieser ist radial nach innen gerichtet und greift im zusammengebauten Zustand des Blutgefäßanschlusses in eine an dem Anschlussteil 4 zwischen dem Absatz 8 und dem Vorsprung 12 ausgebildete ringförmige Ausnehmung 22 ein.

Sowohl an dem Abschnitt 4a des Anschlussteils 4 als auch an dem Klemmbauteil 14 sind über den jeweiligen Umfang verteilt eine Vielzahl von sich radial durch die jeweilige Wandung erstreckenden Durchbrechungen 24 in Form von Langlöchern ausgebildet. Diese Durchbrechungen dienen dazu, ein Verwachsen des Blutgefäßanschlusses mit dem Blutgefäß 6 zu erleichtern und ein Absterben des Gewebes des zwischen dem Anschlussteil 4 und dem Klemmbauteil 14 verklemmten Abschnitts des Blutgefäßes 6 zu verhindern.

Die Befestigungshülse 10 ist in das Innere des Abschnitts 4b einschraubar. Zur Bildung einer Gewindepaarung 26 mit einem an der Innenseite des Abschnitts 4b des Anschlussteil 4 ausgebildeten Innengewinde weist die Befestigungshülse 10 ein Außengewinde auf, das im eingeschraubten Zustand der Befestigungshülse 10 an dem von dem Absatz 8 des Anschlussteils 4 abgewandten Ende der Befestigungshülse 10 angeordnet ist. In axialer Richtung anschließend an das Außengewinde der Befestigungshülse 10 verjüngt sich der Außenquerschnitt der Befestigungshülse 10 absatzförmig, so dass dort zwischen der Innenwandung des erweiterten Abschnitts des Anschlussteils 4 und der wandung der Befestigungshülse 10 ein Zwischen raum 28 entsteht.

Zum Befestigen der Gefäßprothese 2 an dem Anschlussteil 4 wird diese zunächst durch das Innere der Befestigungshülse 10 geführt. An dem im eingeschraubten Zustand dem Absatz 8 zugewandten Ende der Befestigungshülse 10 wird ein über dieses Ende überstehender Abschnitt der Gefäßprothese 2 um das Ende gestülpt und anschließend die Befestigungshülse 10 so weit in das Innere des Abschnitts 4b des Anschlussteils 4 eingeschraubt, bis die Gefäßprothese 2 zwischen dem Absatz 8 und der Stirnseite der Befestigungshülse 10 verklemmt ist. Der überstehende Abschnitt der Gefäßprothese 2 wird hierbei von dem Zwischenraum 28 zwischen der Wandung des Anschlussteils 4 und der Wandung der Befestigungshülse 10 aufgenommen. Der konstante Innenquerschnitt der Gefäßprothese 2 entspricht dem Innenquerschnitt des Anschlussteils 4 im Abschnitt 4a, sodass das Anschlussteil 4 und die darin befestigte Gefäßprothese 2 einen glatten Strömungskanal mit konstanten Innenquerschnitt von dem Blutgefäß 6 zu einem an dem von dem Anschlussteil 4 abgewandten Ende der Gefäßprothese 2 angeschlossenen, in der Zeichnung nicht dargestellten Blutgefäß bilden bzw. nach Resektion eines krankhaften Abschnitts des Blutgefäßes 6 einen Strömungskanal von einem gesunden Abschnitt des Blutgefäßes 6 zu einem weiteren gesunden Abschnitt des Blutgefäßes 6 bilden.

### Bezugszeichenliste

- 2: Gefäßprothese
- 4: Anschlussteil
- 4a, 4b: Abschnitt
- 6: Blutgefäß
- 8: Absatz
- 10: Befestigungshülse
- 12: Vorsprung
- 14: Klemmbauteil
- 14a, 14b: Teil
- 16: Nut
- 18: Vorsprung
- 20: Vorsprung
- 22: Nut
- 24: Durchbrechung
- 26: Gewindepaarung
- 28: Zwischenraum

## Patentansprüche

1. Blutgefäßanschluss für eine Gefäßprothese (2),
mit einem Anschlussteil (4), welches abschnittsweise mit einem rohrförmigen Abschnitt (4a) in ein offenes Ende eines Blutgefäßes (6) einführbar ist und einen Strömungskanal von dem Blutgefäß (6) zu einer an dem Anschlussteil (4) angeschlossenen Gefäßprothese (2) bildet,
mit einem hülsenförmigen Klemmbauteil (14) zur Anordnung außenseitig des rohrförmigen Abschnitts (4a), mit dem das Blutgefäß (6) an dem Anschlussteil (4) befestigbar ist und
mit einer Befestigungshülse (10) zum Fixieren der Gefäßprothese (2)
**dadurch gekennzeichnet,**
**dass** sich ein den Strömungskanal durch das Anschlussteil (4) bildender Hohlraum des Anschlussteils (4) in einem Abschnitt (4b), der sich an den rohrförmigen Abschnitt (4a) anschließt, absatzförmig erweitert,
wobei die Befestigungshülse (10) in den erweiterten Abschnitt (4b) einschraubbar ist und mit dem erweiterten Abschnitt (4b) so dimensioniert ist, dass die Innenwandung der Gefäßprothese (2) mit der Innenwandung des von dem Anschlussteil (4) gebildeten Strömungskanal fluchtet und
wobei der rohrförmige Abschnitt (4a) und das Klemmbauteil (14) jeweils über den Umfang verteilt eine Vielzahl von Durchbrechungen (24) aufweisen.

2. Blutgefäßanschluss nach Anspruch 1, bei dem das Klemmbauteil (14) zweiteilig ausgebildet ist.

3. Blutgefäßanschluss nach Anspruch 2, bei dem die Teile (14a, 14b) des Klemmbauteils (14) zur Bildung einer Rastverbindung miteinander ausgebildet sind.

4. Blutgefäßanschluss nach einem der vorangehenden Ansprüche, bei dem an dem Klemmbauteil (14) zumindest eine, sich um den Außenumfang des Klemmbauteils (14) erstreckende Nut (16) ausgebildet ist.

5. Blutgefäßanschluss nach einem der Ansprüche 2 bis 4, bei dem die Teile (14a, 14b) des Klemmbauteils (14) mittels eines Scharniers verbunden sind.

6. Blutgefäßanschluss nach einem der vorangehenden Ansprüche, bei dem das Klemmbauteil (14) und das Anschlussteil (4) formschlüssig verbindbar sind.

7. Blutgefäßanschluss nach Anspruch 6, bei dem das Klemmbauteil (14) zumindest einen radial nach innen gerichteten Vorsprung (20) aufweist, welcher zum Eingriff in zumindest eine an der Außenseite des Anschlussteils (4) ausgebildete Ausnehmung (22) vorgesehen ist.

8. Blutgefäßanschluss nach einem der vorangehenden Ansprüche, bei dem außenseitig des in das Blutgefäß (6) einführbaren Abschnitts (4a) des Anschlussteils (4) zumindest ein radial nach außen gerichteter Vorsprung ausgebildet ist, der sich vorzugsweise um den gesamten Umfang des Anschlussteils (4) erstreckt.

9. BlutgefäBanschluss nach einem der vorangehenden Ansprüche, bei dem sich der Außenquerschnitt des Anschlussteils (4) im Bereich des rohrförmigen Abschnitts (4a) in Richtung dessen freien Endes verjüngt.

10. Blutgefäßanschluss nach einem der vorangehenden Ansprüche, bei dem die Befestigungshülse (10) in axialer Richtung derart dimensioniert ist, dass eine durch die Befestigungshülse (10) geführte Gefäßprothese 2), welche das im eingeschraubten Zustand in das Anschlussteil (4) eingreifende Ende der Befestigungshülse (10) überlappt, zwischen diesem Ende der Befestigungshülse (10) und dem Absatz (8) des Anschlussteils (4) verklemmbar ist.

11. Blutgefäßanschluss nach einem der vorangehenden Ansprüche, welcher aus Polyethylenterephthalat ausgebildet ist.

## Claims

1. A blood vessel connection for a vessel prosthesis (2),
with a connection part (4) which in sections with an annular section (4a) can be introduced into an open end of a blood vessel (6) and forms a flow channel from the blood vessel (6) to a vessel prosthesis (2) connected on the connection part (4),
with a sleeve-like clamping component (14) for the arrangement on the outside of the tubular section (4a) and with which the blood vessel (6) can be fastened on the connection part (4) and
with a fastening sleeve (10) for fixing the vessel prosthesis (2)
**characterised in that**
a cavity of the connection part (4) which forms the flow channel through the connection part (4) is widened in a shoulder-like manner in a section (4b) connecting to the tubular section (4a),
wherein the fastening sleeve (10) can be screwed into the widened section (4b) and with the widened section is dimensioned such that the inner wall of the vessel prosthesis (2) is aligned with the inner wall of the flow channel formed by the connection part (4) and
wherein the tubular section (4a) and the clamping component (14) comprise a multitude of openings (24) in each case distributed over the periphery.

2. A blood vessel connection according to claim 1, with which the clamping component (14) is designed of two parts.

3. A blood vessel connection according to claim 2, with which the parts (14a, 14b) of the clamping component (14) are designed for forming a locking connection with one another.

4. A blood vessel connection according to one of the preceding claims, with which at least one groove (16) extending around the outer periphery of the clamping component (14), is formed on the clamping component (14).

5. A blood vessel connection according to one of the claims 2 to 4, with which the parts (14a, 14b) of the clamping component (14) are connected by way of a hinge.

6. A blood vessel connection according to one of the preceding claims, with which the clamping component (14) and the connection part (4) can be connected with a positive fit.

7. A blood vessel connection according to claim 6, with which the clamping component (14) comprises at least one radially inwardly directed projection (20) which is envisaged for engagement into at least one recess (22) which is formed on the outer side of the connection part (4).

8. A blood vessel connection according to one of the preceding claims, with which at least one radially outwardly directed projection which extends preferably around the whole periphery of the connection part (4), is formed on the outer side of the section (4a) of the connection part (4) which can be introduced into the blood vessel (6).

9. A blood vessel connection according to one of the preceding claims, with which the outer cross section of the connection part (4), in the region of the tubular section (4a), tapers in the direction of its free end.

10. A blood vessel connection according to one of the preceding claims, with which the fastening sleeve (10) is dimensioned in the axial direction in a manner such that a vessel prosthesis (2) which is led through the fastening sleeve (10) and which overlaps the end of the fastening sleeve (10) which engages into the connection part (4) in the screwed-in condition, can be clamped between this end of the fastening sleeve (10) and the shoulder (8) of the connection part (4).

11. A blood vessel connection according to one of the preceding claims, which is formed of polyethylene terephthalate.

## Revendications

1. Raccord de vaisseau sanguin pour prothèse vasculaire (2), comprenant
une partie de raccord (4) qui peut être introduite en partie, par un segment tubulaire (4a), dans une extrémité ouverte d'un vaisseau sanguin (6) et qui forme un canal d'écoulement allant du vaisseau sanguin (6) à une prothèse vasculaire (2) raccordée à la partie de raccord (4),
un élément de serrage (14) en forme de douille destiné à être placé à l'extérieur du segment tubulaire (4a), au moyen duquel le vaisseau sanguin (6) peut être fixé à la partie de raccord (4), et
une douille de fixation (10) servant à fixer la prothèse vasculaire (2),
**caractérisé**
**en ce qu'**une cavité de la partie de raccord (4) qui forme le canal d'écoulement à travers la partie de raccord (4) s'élargit, en formant un épaulement, en un segment (4b) qui se raccorde au segment tubulaire (4a),
la douille de fixation (10) pouvant être vissée dans le segment élargi (4b) et étant dimensionnée relativement à la partie élargie (4b) de façon telle que la paroi intérieure de la prothèse vasculaire (2) est dans l'alignement de la paroi intérieure du canal d'écoulement formé par la partie de raccord (4), et
le segment tubulaire (4a) et l'élément de serrage (14) présentant chacun une pluralité de fenêtres (24) réparties sur la circonférence.

2. Raccord de vaisseau sanguin selon la revendication 1, dans lequel l'élément de serrage (14) est exécuté en deux parties.

3. Raccord de vaisseau sanguin selon la revendication 2, dans lequel les parties (14a, 14b) de l'élément de serrage (14) sont configurées pour former ensemble un assemblage à encliquetage.

4. Raccord de vaisseau sanguin selon l'une des revendications précédentes, dans lequel est formée sur l'élément de serrage (14) au moins une gorge (16) qui s'étend autour de la périphérie extérieure de l'élément de serrage (14).

5. Raccord de vaisseau sanguin selon l'une des revendications 2 à 4, dans lequel les parties (14a, 14b) de l'élément de serrage (14) sont assemblées au moyen d'une charnière.

6. Raccord de vaisseau sanguin selon l'une des revendications précédentes, dans lequel l'élément de serrage (14) et la partie de raccord (4) peuvent être assemblés par engagement mécanique.

7. Raccord de vaisseau sanguin selon la revendication 6, dans lequel l'élément de serrage (14) présente au moins une saillie (20) dirigée radialement vers l'intérieur, qui est prévue pour s'engager dans au moins un évidement (22) formé sur le côté extérieur de la partie de raccord (4).

8. Raccord de vaisseau sanguin selon l'une des revendications précédentes, dans lequel, sur le côté extérieur du segment (4a) de la partie de raccord (4) qui peut être engagé dans le vaisseau sanguin (6), est formée au moins une saillie dirigée radialement vers l'extérieur, qui s'étend de préférence sur tout le périmètre de la partie de raccord (4).

9. Raccord de vaisseau sanguin selon l'une des revendications précédentes, dans lequel la section transversale extérieure de la partie de raccord (4) se rétrécit en direction de son extrémité libre dans la région du segment tubulaire (4a).

10. Raccord de vaisseau sanguin selon l'une des revendications précédentes, dans lequel la douille de fixation (10) est dimensionnée dans la direction axiale de telle manière qu'une prothèse vasculaire (2) guidée à travers la douille de fixation (10), qui recouvre l'extrémité de la douille de fixation (10) qui est engagée dans la partie de raccord (4) dans son état vissé, peut être serrée entre cette extrémité de la douille de fixation (10) et l'épaulement (8) de la partie de raccord (4).

11. Raccord de vaisseau sanguin selon l'une des revendications précédentes, qui est fabriqué en téréphtalate de polyéthylène.
